(19) **Europäisches Patentamt / European Patent Office / Office européen des brevets**

(11) **EP 1 815 837 A1**

(12) # EUROPEAN PATENT APPLICATION

published in accordance with Art. 158(3) EPC

(43) Date of publication:
**08.08.2007 Bulletin 2007/32**

(51) Int Cl.:
***A61K 6/00*** *(2006.01)*

(21) Application number: **05815873.4**

(22) Date of filing: **11.11.2005**

(86) International application number:
**PCT/ES2005/000609**

(87) International publication number:
**WO 2006/058933 (08.06.2006 Gazette 2006/23)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **26.11.2004 ES 200402848**

(71) Applicant: **Laboratorios Kin, S.A.**
**08018 Barcelona (ES)**

(72) Inventors:
• **SANCHO RIERA, Enriqueta**
  **E-08029 Barcelona (ES)**
• **ARGUDO CARRERAS, Eva**
  **E-08980 Sant Feliu de Llobregat (ES)**

(74) Representative: **Curell Aguilà, Mireia et al**
**Dr. Ing. M. Curell Sunol I.I.S.L.**
**Passeig de Gràcia 65 bis**
**08008 Barcelona (ES)**

(54) **MOUTHWASH COMPRISING CALCIUM AND FLUORINE IONS**

(57) Mouthwash containing fluorine and calcium ions. The mouthwash has a pH value between 4.5 and 6.5 and does not contain any calcium ion sequestrants having a chelating constant with the calcium ion greater than $10^4$. This way a mouthwash can be obtained having a high stability over time and a high therapeutic effectiveness.

EP 1 815 837 A1

**Description**

Field of the invention

The invention relates to a mouthwash containing fluorine and calcium ions.

Prior art

In compositions for treating the oral cavity it is common to add fluorine ions. It is also common to add calcium ions. Various documents have described the possibility of preparing compositions for treating the oral cavity that contain both ions, such as for example in "Improving the performance of sodium monofluorophosphate to protect enamel against acid" G.C. Forward et.al., Caries Research 13: 61-67 (1979) and in FR 1.186.136.

However both ions have a strong tendency to combine together causing a precipitate. This problem has also been analysed in various documents, such as FR 1.186.136 mentioned above, EP 89.136 A2, and US 6.863.882. The formation of a large amount of precipitate reduces the effectiveness of the composition, and in these documents the addition of a calcium ion sequestrant is proposed to reduce the amount of precipitate.

However, the formation of a precipitate, even in small amounts, has an unfavourable aesthetic effect in mouthwashes, which must be totally transparent, regardless of whether or not they are coloured, and which must maintain their transparency on a long term basis, because they are products that may have a "useful life" of several months, or even years.

Summary of the invention

The object of the invention is to overcome these drawbacks. This aim is achieved by means of a mouthwash of the type indicated at the beginning characterised in that it has a pH between 4.5 and 6.5 and does not contain any calcium ion sequestrants having a chelating constant with the calcium ion greater than $10^4$. In fact, one way of preventing the calcium fluoride ($CaF_2$) precipitate is by adding a calcium ion sequestrant. This way the amount of free calcium ion in the solution is less, whereby the formation of a precipitate is reduced and even eliminated. However, a further consequence of adding a calcium ion sequestrant is that the mouthwash is less effective, because the smaller amount of free calcium ion makes the solution less effective in therapeutical terms. Therefore merely adding a sequestrant agent is not enough to obtain an optimised mouthwash in terms of its therapeutic effectiveness. Moreover, it has been observed that the solution's pH value also has an important effect on the formation of the precipitate. The inventors have discovered that a solution having a pH between 4.5 and 6.5 is virtually stable in the sense that the calcium fluoride does not precipitate, even after long periods of time. Long term stability is important, because it is common for precipitates to form several months after production, which means that they are noticeable when the end user already has the product in his/her home and/or that they are noticeable in the establishment where they are sold to the public. Solutions having a pH between 4.5 and 6.5 do not require the addition of calcium ion sequestrants with a high chelating constant. So, calcium ion sequestrants having chelating constants below or equivalent to $10^4$ are enough to obtain stable solutions in the sense that the calcium fluoride does not precipitate even on a long term basis (a long term basis being understood to be six months or more). By way of a reference, it can be mentioned that EDTA is a conventional calcium ion sequestrant, with a constant approximately equivalent to $5 \times 10^{10}$. Adding EDTA to a solution with fluorine and calcium ions clearly prevents them from precipitating, but it binds the calcium ions to such an extent that the solution loses a large degree of its effectiveness.

None of the documents mentioned above analyses the problem of long term stability. Moreover, the analysed compositions are usually toothpastes (in which a certain amount of precipitating is not noticeable) or bicomponent mouthwashes (wherein both ions remain together for just a few minutes). In fact, document US 6.863.882 considers as acceptable a precipitation of up to 20% of the originally provided fluorine ion. These values are totally unacceptable for a mouthwash, as it would have an unacceptable cloudy appearance. None of the preceding documents describes a composition having a calcium ion sequestrant with a low chelating constant and which guarantees long term stability. The inventors have discovered that by adjusting the pH value within an exact interval (between 4.5 and 6.5) it is possible to obtain a composition that is stable on a long term basis using a calcium ion sequestrant with a low chelating constant. None of the cited documents refers to this pH interval and, in fact, the examples described therein all have a pH greater or equivalent to 7.

In this specification and claims the term "chelating constant" is used to refer to the balance constant of the complex formed by the calcium ion (Ca) and the sequestrant (Q), which is defined by the following formula:

$$K_{eq} = [CaQ]/[Ca][Q]$$

Preferably the mouthwash does not contain any calcium ion sequestrants having a chelating constant with the calcium ion greater than $4\times10^3$. In fact, preferably the mouthwash contains citric acid, which means the solution's pH can be adjusted in a particularly suitable way. The citric acid is, also, a calcium ion sequestrant although it has a very low chelating constant. The chelating constants usually have values that vary according to various parameters. However, it can be considered that the chelating constant of citric acid is lower than $4x10^3$. In this respect, advantageously the mouthwash does not contain any calcium ion sequestrants having a chelating constant with the calcium ion greater than $3.2x10^3$, which is the value of the chelating constant of the citric acid indicated in the bibliography (CRC Handbook of Food Additives, Thomas E. Furia 2nd ed. 1972 revised 28 May 2001). In other words, it is particularly advantageous that the mouthwash does not contain any calcium ion sequestrants having a chelating constant with the calcium ion greater than citric acid. In fact this is the ideal combination which allows a maximum amount of free calcium ion in the solution without the calcium fluoride precipitating even after long periods of time (over six months). The citric acid, with its soft binding action, makes it possible to combine the effect of a pH value controlled between 4.5 and 6.5 and a slight prevention of the precipitation by binding the calcium ions.

The inventors have observed that with a pH value lower or equivalent to 7 apparently precipitation no longer takes place. However, this is certain only over a short period of time and, after a few weeks, a noticeable precipitation starts to occur. On the contrary, if the pH value is lower or equivalent to 6.5, then the solution is stable on a long term basis, so that after more than 6 months no noticeable precipitation has occurred.

Preferably the mouthwash has a concentration of calcium ions between $1.9x10^{-4}$ and 0.3 grams per 100 ml of solution, preferably $9.5x10^{-3}$g/100ml.

Advantageously the mouthwash has a concentration of fluorine ions between $5x10^{-3}$ and 1.05 g per 100 ml of solution, preferably 0.023g/100ml.

Detailed description of some embodiments of the invention

Some examples are described below which indicate the effectiveness of the invention. In the following examples the values indicated for the various solution components are expressed in grams per 100 ml of solution.

Example 1:

Table 1 shows three solution examples containing a fluorine ion and a calcium ion. The fluorine ion is provided in the form of sodium monofluorophosphate, and the calcium ion is provided in the form of calcium glycerophosphate, both in this particular example and in the following examples. As it can be appreciated in Table 1, a calcium fluoride precipitate is always obtained that clouds the solution.

TABLE 1

|  | Case 1 | Case 2 | Case 3 |
|---|---|---|---|
| Sodium monofluorophosphate | 0.17 | 0.17 | 0.17 |
| Calcium glycerophosphate | 0.13 | 0.065 | 0-033 |
| Result | Cloudy | Cloudy | Cloudy |

Example 2:

Table 2 shows four solution examples containing a fluorine ion and a calcium ion and which, also, include various percentages of EDTA. The EDTA is a known calcium ion sequestrant with a high chelating constant (approximately equivalent to $5x10^{10}$). As can be seen in Table 2, the EDTA prevents the formation of the precipitate (except where the EDTA contributions are very low) because the EDTA binds the calcium ion. However its high chelating constant means that neither is the calcium ion available for its therapeutic function.

TABLE 2

|  | Case 1 | Case 2 | Case 3 | Case 4 |
|---|---|---|---|---|
| Sodium monofluorophosphate | 0.17 | 0.17 | 0.17 | 0.17 |

(continued)

|  | Case 1 | Case 2 | Case 3 | Case 4 |
|---|---|---|---|---|
| Calcium glycerophosphate | 0.13 | 0.13 | 0.13 | 0.13 |
| EDTA | 0.1 | 0.2 | 0.5 | 0.05 |
| Result | Transparent | Transparent | Transparent | Cloudy |

Example 3:

Eight variants of a mouthwash formula have been prepared, containing fluorine ions and calcium ions. The eight variants are all the same, except that their pH values have been adjusted to different values, in particular to the values 4, 4-5, 5, 5.5, 6, 6.5, 7 and 7-5- In each case the pH is adjusted by the action of citric acid. The citric acid has been added basically to adjust the pH value. Although citric acid is also a calcium ion sequestant, its low chelating constant (below $10^4$, in other words, about 6 orders of magnitude less than the chelating constant of EDTA) means that the therapeutic effectiveness of the calcium ion is not affected. The formula was as follows:

| | |
|---|---|
| Sodium monofluorophosphate | 0.170 % |
| Calcium glycerophosphate | 0.050 %. |
| Wetting agents | 2.5-10% |
| Citric acid ad. pH 4, 4.5, 5, 5.5, 6, 6.5, 7 and 7.5 | |
| Purified water ad. | 100.000 ml |

The wetting agents can be, for example, glycerine. The formula can contain, optionally, aromas, sweeteners (such a sodium saccharine, xylitol, sorbitol, sodium cyclamate, acesulfame potassium), preservatives (such as methyl, ethyl or propyl parahydroxybenzoates), etc.

The formula with a pH value equivalent to 7.5 precipitated after about two weeks. The formula with a pH value equivalent to 7 began to show signs of precipitating after four weeks, and these signs were more noticeable after six weeks. All the other samples remained stable without any signs of precipitation for more than six months. Therefore the pH value equivalent to 6.5 seems to be the limit of what can be considered a product with a virtually indefinite stability.

**Claims**

1. Mouthwash containing fluorine and calcium ions, **characterised in that** it has a pH value between 4.5 and 6.5 and does not contain any calcium ion sequestrants having a chelating constant with the calcium ion greater than $10^4$.

2. Mouthwash according to claim 1, **characterised in that** it does not contain any calcium ion sequestrants having a chelating constant with the calcium ion greater than $4 \times 10^3$.

3. Mouthwash according to claim 1, **characterised in that** it does not contain any calcium ion sequestrants having a chelating constant with the calcium ion greater than $3'2 \times 10^3$, particularly a chelating constant with the calcium ion greater than citric acid.

4. Mouthwash according to any of the claims 1 to 3, **characterised in that** it has a concentration of calcium ions between $1.9 \times 10^{-4}$ and 0.3 grams per 100 ml of solution, preferably $9.5 \times 10^{-3}$ g/100 ml.

5. Mouthwash according to any of the claims 1 to 4, **characterised in that** it has a concentration of fluorine ions between $5 \times 10^{-3}$ and 1.05 g per 100 ml of solution, preferably 0.023 g/100 ml.

# EP 1 815 837 A1

<table>
<tr><td align="center"><strong>INTERNATIONAL SEARCH REPORT</strong></td><td>International application No.<br>PCT/ ES 2005/000609</td></tr>
</table>

A. CLASSIFICATION OF SUBJECT MATTER

### See additional sheet

According to International Patent Classification (IPC) or to both national classification and IPC

B. FIELDS SEARCHED

Minimum documentation searched (classification system followed by classification symbols)

A61K

Documentation searched other than minimum documentation to the extent that such documents are included in the fields searched

Electronic data base consulted during the international search (name of data base and, where practicable, search terms used)

CIBEPAT,EPODOC, WPI, NPL, EMBASE, BIOSIS, MEDLINE, HCAPLUS

C. DOCUMENTS CONSIDERED TO BE RELEVANT

| Category* | Citation of document, with indication, where appropriate, of the relevant passages | Relevant to claim No. |
|---|---|---|
| A | **EP 089136 A (THE PROCTER & GAMBLE COMPANY) 21.09.1983, examples, claims.** | 1-5. |
| A | **US 2003124066 A (DLXON, J.R. ET AL) 03.07.2003, page 1, parr. 15, 16 and 17,page 4, parr. 49, page 5, parr. 62, examples 1-9,claims.** | 1-5. |
| A | **US 4925655 A (SMIGEL ET AL) 15.05.1990, the whole document.** | 1-5. |

☐ Further documents are listed in the continuation of Box C.    ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **08 FEB 2006 (08.02.06)** | **20 FEB 2006 (20.02.06)** |
| Name and mailing address of the ISA/<br><br>**S.P.T.O.** | Authorized officer |
| Facsimile No. | Telephone No. |

Form PCT/ISA/210 (second sheet) (July 1992)

**INTERNATIONAL SEARCH REPORT**
Information on patent family members

International Application No

PCT/ ES 2005/000609

| Patent document cited in search report | Publication date | Patent familiy member(s) | Publication date |
|---|---|---|---|
| EP 0089136 A | 21.09.1983 | FI 830786 A<br>AU 1219983 A<br>JP 58219107 A<br>GR 77131 A | 11.09.1983<br>15.09.1983<br>20.12.1983<br>07.09.1984 |
| US 2003124066 A | 03.07.2003 | US 6863882 B<br>WO 03059304 A<br>CA 2469431 A<br>EP 1461006 A<br>CN 1610535 A | 08.03.2005<br>24.07.2003<br>24.07.2003<br>29.09.2004<br>27.04.2005 |
| US 4925655 A | 15.05.1990 | CA 2010896 A<br>WO 9115190 A | 26.08.1991<br>17.10.1991 |

**INTERNATIONAL SEARCH REPORT**
Information on patent family members

International Application No

PCT/ ES 2005/000609

**IPC 8**

*A61K 8/69 (2006.01)*
*A61Q 11/00 (2006.01)*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 1186136
- EP 89136 A2

- US 6863882 B

**Non-patent literature cited in the description**

- **G.C. FORWARD.** Improving the performance of sodium monofluorophosphate to protect enamel against acid. *Caries Research,* 1979, vol. 13, 61-67

- **THOMAS E. FURIA.** CRC Handbook of Food Additives. 1972